# EUROPEAN PATENT APPLICATION

(11) **EP 4 112 648 A1**
(43) Date of publication of application: **04.01.2023**
(21) Application number: 20920803.2
(22) Date of filing: 18.09.2020
(51) Int. Cl.: C07K 19/00, C12N 15/62, G01N 33/68, A61K 39/215, C12N 7/04

(54) **ANTI-SARS-COV-2 INFECTION PROTEIN AND VACCINE**

(30) Priority: 24.02.2020 CN 202010113054
(71) Applicant: West Vac Biopharma Co., Ltd., Chengdu, Sichuan 610000 (CN)
(72) Inventor: WEI, Xiawei, Chengdu, Sichuan 610065 (CN); LU, Guangwen, Chengdu, Sichuan 610065 (CN); WANG, Wei, Chengdu, Sichuan 610065 (CN); YANG, Jinliang, Chengdu, Sichuan 610065 (CN); YANG, Li, Chengdu, Sichuan 610065 (CN); LI, Jiong, Chengdu, Sichuan 610065 (CN); YANG, Jingyun, Chengdu, Sichuan 610065 (CN); WEI, Yuquan, Chengdu, Sichuan 610065 (CN); WANG, Zhenling, Chengdu, Sichuan 610065 (CN); ZHAO, Zhiwei, Chengdu, Sichuan 610065 (CN); SHEN, Guobo, Chengdu, Sichuan 610065 (CN)
(74) Representative: Gulde & Partner
(86) International application number: PCT/CN2020/116109
(87) International publication number: WO 2021/169255

(57) **Abstract**

An anti-SARS-CoV-2 infection protein and vaccine. The protein contains a structural domain bound to an angiotensin converting enzyme 2 receptor in an S protein of SARS-CoV-2. A vaccine for preventing and/or treating the SARS-CoV-2 infection contains the anti-SARS-CoV-2 infection protein, and pharmaceutically acceptable auxiliary materials or auxiliary components.

## Description

### Field of the Invention

The present invention relates to an anti- SARS-CoV-2-infection protein and vaccine, and belongs to the field of medicine.

### Background of the Invention

SARS-CoV-2 is a novel beta coronavirus (β-CoV) named by the World Health Organization (WHO). The virus has an envelope and is present in round or oval (often pleomorphic) particles, with a diameter of 60-140nm. With obviously different gene characteristics from SARS-CoV and MERS-CoV, it is an unprecedented human novel coronavirus branch. Bats may be the natural host of SARS-CoV-2, and pangolins have also been suggested as a possible animal source of the virus. At present, the novel coronavirus SARS-CoV-2 has already infected tens of thousands of people, but there are still no definitely effective antiviral drugs for prevention and treatment. Therefore, the research and development of the related virus vaccine is significantly important for the disease prevention and treatment.

Main structural proteins of SARS-CoV-2 include spike (S), envelop (E), membrane (M) and nucleocapsid (N), among which S protein plays the key role in virus infection and virulence. Angiotensin-converting enzyme 2 (ACE2) is the functional receptor of SARS coronavirus, while recent research shows that SARS-CoV-2 enters the host cell through binding with the ACE2 receptor for virus infection and replication. SARS-CoV-2 S protein consists of two domains, S1 and S2. S 1 protein, as the receptor-binding domain (RBD) that binds with the ACE2 receptor, is responsible for binding of virus with the host cell receptor, fusion with the cell membrane, and virus invasion and infection.

### Summary of the Invention

The present invention is intended to solve one of the technical problems of the prior art. Therefore, the purpose of the present invention is to provide an anti-SARS-CoV-2-infection protein. Another purpose of the present invention is to provide a vaccine containing the protein for SARS-CoV-2 infection prevention and/or treatment.

The present invention provides an anti-SARS-CoV-2-infection protein, which contains a domain that binds with the angiotensin-converting enzyme 2 (ACE2) receptor as contained in the SARS-CoV-2 S protein.

Furthermore, the structure basis of the domain is the 319th-541th amino acids of RBD in S protein and the 319th amino acid is dispensable.

Furthermore, the amino acid sequence of the domain is SEQ ID No.1 or SEQ ID No.2.

Furthermore, the amino acid sequence of the protein is at least one of the SEQ ID No.1, SEQ ID No.2, SEQ ID No.3, and SEQ ID No.4.
SEQ ID No.1: 
RVQPTESIVRFPNITNLCPFGEVFNATRFASVYAWNRKRISNCVADYSVLYNSA
SFSTFKCYGVSPTKLNDLCFTNVYADSFVIRGDEVRQIAPGQTGKIADYNYKL
PDDFTGCVIAWNSNNLDSKVGGNYNYLYRLFRKSNLKPFERDISTEIYQAGST
PCNGVEGFNCYFPLQSYGFQPTNGVGYQPYRVVVLSFELLHAPATVCGPKKST
NLVKNKCVNF
SEQ ID No.2: 
VQPTESIVRFPNITNLCPFGEVFNATRFASVYAWNRKRISNCVADYSVLYNSAS
FSTFKCYGVSPTKLNDLCFTNVYADSFVIRGDEVRQIAPGQTGKIADYNYKLP
DDFTGCVIAWNSNNLDSKVGGNYNYLYRLFRKSNLKPFERDISTEIYQAGSTP
CNGVEGFNCYFPLQSYGFQPTNGVGYQPYRVVVLSFELLHAPATVCGPKKST
NLVKNKCVNF
SEQ ID No.3: 
RVQPTESIVRFPNITNLCPFGEVFNATRFASVYAWNRKRISNCVADYSVLYNSA
SFSTFKCYGVSPTKLNDLCFTNVYADSFVIRGDEVRQIAPGQTGKIADYNYKL
PDDFTGCVIAWNSNNLDSKVGGNYNYLYRLFRKSNLKPFERDISTEIYQAGST
PCNGVEGFNCYFPLQSYGFQPTNGVGYQPYRVVVLSFELLHAPATVCGPKKST
NLVKNKCVNFNFNG
SEQ ID No.4:

VQPTESIVRFPNITNLCPFGEVFNATRFASVYAWNRKRISNCVADYSVLYNSAS
FSTFKCYGVSPTKLNDLCFTNVYADSFVIRGDEVRQIAPGQTGKIADYNYKLP
DDFTGCVIAWNSNNLDSKVGGNYNYLYRLFRKSNLKPFERDISTEIYQAGSTP
CNGVEGFNCYFPLQSYGFQPTNGVGYQPYRVVVLSFELLHAPATVCGPKKST
NLVKNKCVNFNFNG

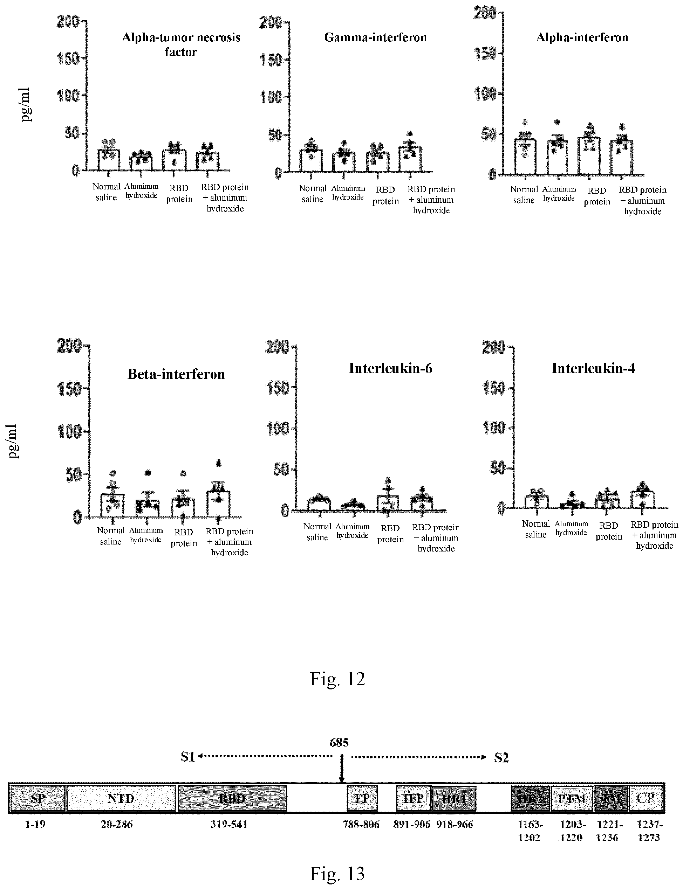

Extracellular domain composition of the SARS-CoV-2 S protein is shown in Fig. 13; wherein, SP stands for the signal peptide, NTD the N-terminal domain, RBD the receptor-binding domain, FP the fusion peptide, IFP the internal fusion peptide, HR1 the heptad repeat 1, HR2 the heptad repeat 2, PTM the proximal transmembrane domain, and TM the transmembrane domain.

The protein shown in SEQ ID No.1 is an anti- SARS-CoV-2-infection drug designed based on the RBD (the 319th-541th amino acids). Protein tags are inserted in the amino acid sequence of the protein herein, and the 1st amino acid R in the sequence is dispensable, as shown in SEQ ID No.2.

Preferably, the protein as shown in SEQ ID No.3 has four additional amino acids NFNG after the 541th amino acid (that is, there are actually the 319th-545th amino acids in the RBD), which can enhance the stability of the anti- SARS-CoV-2-infection protein described in the present invention. Protein tags are inserted in the amino acid sequence of the protein herein, and the 1st amino acid R in the sequence is dispensable, as shown in SEQ ID No.4.

Furthermore, the 8xHis protein tag is fused at the C-terminal, which can felicitate the protein purification.

The present invention provides the precursor of the protein which links the anti-SARS-CoV-2-infection protein with a signal peptide and/or protein tag.

Preferably, the protein tag is at least one of the histidine tag, thioredoxin tag, glutathione transferase tag, ubiquitin-like modified protein tag, maltose-binding protein tag, c-Myc protein tag, Avi tag protein tag, and nitrogen source utilization substance A protein tag.

Furthermore, the precursor also links the anti-SARS-CoV-2-infection protein with a protease recognition area for protein tag removal.

Preferably, the protease is at least one of the enterokinase, TEV protease, thrombin, coagulation factor Xa, carboxypeptidase A, and rhinovirus 3c protease.

Furthermore, the amino acid sequence is at least one of the SEQ ID No.5, SEQ ID No.6, SEQ ID No.7 and SEQ ID No.14.
SEQ ID No.5 (Insect cell signal peptide + S protein + His tag amino acid sequence): 
MLLVNQSHQGFNKEHTSKMVSAIVLYVLLAAAAHSAFAADVQPTESIVRFPNI
TNLCPFGEVFNATRFASVYAWNRKRISNCVADYSVLYNSASFSTFKCYGVSPT
KLNDLCFTNVYADSFVIRGDEVRQIAPGQTGKIADYNYKLPDDFTGCVIAWNS
NNLDSKVGGNYNYLYRLFRKSNLKPFERDISTEIYQAGSTPCNGVEGFNCYFP
LQSYGFQPTNGVGYQPYRVVVLSFELLHAPATVCGPKKSTNLVKNKCVNFNF
NGHHHHHHHH
SEQ ID No.6 (Insect cell signal peptide + Escherichia coli Trx (thioredoxin) + S protein RBD amino acid sequence): 
MLLVNQSHQGFNKEHTSKMVSAIVLYVLLAAAAHSAFAADSDKIIHLTDDSFD
TDVLKADGAILVDFWAEWCGPCKMIAPILDEIADEYQGKLTVAKLNIDQNPGT
APKYGIRGIPTLLLFKNGEVAATKVGALSKGQLKEFLDANLAGSGSGHMHHH
HHHSSGDDDDKVQPTESIVRFPNITNLCPFGEVFNATRFASVYAWNRKRISNCV
ADYSVLYNSASFSTFKCYGVSPTKLNDLCFTNVYADSFVIRGDEVRQIAPGQT
GKIADYNYKLPDDFTGCVIAWNSNNLDSKVGGNYNYLYRLFRKSNLKPFERD
ISTEIYQAGSTPCNGVEGFNCYFPLQSYGFQPTNGVGYQPYRVVVLSFELLHAP
ATVCGPKKSTNLVKNKCVNFNFNG
SEQ ID No.7 (Insect cell signal peptide + insect Trx (thioredoxin) + S protein RBD amino acid sequence): 
MLLVNQSHQGFNKEHTSKMVSAIVLYVLLAAAAHSAFAADSIHIKDSDDLKN
RLAEAGDKLVVIDFMATWCGPCKMIGPKLDEMANEMSDCIVVLKVDVDECE
DIATEYNINSMPTFVFVKNSKKIEEFSGANVDKLRNTIIKLKLAGSGSGHMHH
HHHHSSGDDDDKVQPTESIVRFPNITNLCPFGEVFNATRFASVYAWNRKRISN
CVADYSVLYNSASFSTFKCYGVSPTKLNDLCFTNVYADSFVIRGDEVRQIAPG
QTGKIADYNYKLPDDFTGCVIAWNSNNLDSKVGGNYNYLYRLFRKSNLKPFE
RDISTEIYQAGSTPCNGVEGFNCYFPLQSYGFQPTNGVGYQPYRVVVLSFELL
 HAPATVCGPKKSTNLVKNKCVNFNFNG
SEQ ID No.14 (Human IL-6 protein signal peptide + 8xHis signal peptide + EK restriction enzyme cutting site +RBD 320-545 226aa amino acid sequence):

MNSFSTSAFGPVAFSLGLLLVLPAAFPAPHHHHHHHHDDDDKVQPTESIVRFP
NITNLCPFGEVFNATRFASVYAWNRKRISNCVADYSVLYNSASFSTFKCYGVSP
TKLNDLCFTNVYADSFVIRGDEVRQIAPGQTGKIADYNYKLPDDFTGCVIAW
NSNNLDSKVGGNYNYLYRLFRKSNLKPFERDISTEIYQAGSTPCNGVEGFNCY
FPLQSYGFQPTNGVGYQPYRVVVLSFELLHAPATVCGPKKSTNLVKNKCVNF
NFNG

The present invention provides the use of the protein and/or the precursor in preparing the SARS-CoV-2 infection prevention and/or treatment drugs.

The present invention provides a vaccine for SARS-CoV-2 infection prevention and/or treatment, which comprises the protein and/or the precursor as well as the pharmaceutically acceptable excipient or auxiliary ingredient.

Furthermore, the auxiliary ingredient is the immunologic adjuvant.

Preferably, the immunologic adjuvant is at least one of the aluminum salt, calcium salt, plant saponin, plant polysaccharide, monophosphate-lipid A, murinyl dipeptide, murinyl tripeptide, squalene oil-in-water emulsion (MF59), recombinant cholera toxin (rCTB), GM-CSF cytokine, lipid, cationic liposome material, and CpG ODN (nucleotide sequence with non-methylated cytosine and guanine dinucleotides as the core sequence, and synthetic CpG).

Furthermore, the aluminum salt is at least one of the aluminum hydroxide and alum.

Furthermore, the calcium salt is tricalcium phosphate.

Furthermore, the plant saponin is QS - 21 or ISCOM.

Furthermore, the plant polysaccharide is astragalus polysaccharide (APS).

Furthermore, the lipid is at least one of the phosphatidyl ethanolamine (PE), phosphatidyl choline (PC), cholesterol (Chol), and dioleylphosphatidyl ethanolamine (DOPE).

Furthermore, the cationic liposome material is at least one of the (2,3-Dioleoyloxy-propyl)-trimethylammonium-chloride (DOTAP), N-[1-2,3-dioleyoxy, propyl]-n,n,n-trimethylammonium chloride (DOTMA), cationic cholesterol (DC-Chol), trifluoroacetic acid dimethyl-2, 3-dioleoxy propyl-2-(2-spermine formyl amino) ethyl ammonium (DOSPA), dodecyl trimethyl ammonium bromide (DTAB), tetradecyl trimethyl ammonium bromide (TTAB), cetyl-methyl-ammoniumbromide (CTAB), and dimethyldioctadecylammonium bromide (DDAB).

Furthermore, the vaccine is an injection preparation.

Preferably, the vaccine is an intramuscular injection preparation.

The present invention provides a polynucleotide, which encodes the protein and/or the precursor.

Furthermore, the nucleotide sequence of the polynucleotide is at least one of the SEQ ID No.8, SEQ ID No.9, SEQ ID No. 10, SEQ ID No.11, SEQ ID No.12 and SEQ ID No. 13.
SEQ ID No.8 (Insect cell signal peptide + S protein RBD + His tag optimized corresponding nucleotide sequence): 
GGATCCATGCTGCTGGTCAACCAATCTCATCAGGGCTTCAACAAAGAACAT
ACTTCAAAAATGGTCTCCGCTATCGTGCTGTACGTGCTCCTCGCTGCTGCTG
CTCACTCTGCTTTCGCTGCTGACGAATTCAGGGTGCAGCCAACCGAATCTA
TCGTCAGATTCCCAAACATCACTAACCTGTGCCCTTTCGGAGAGGTGTTCA
ACGCTACCAGGTTCGCCAGCGTCTACGCTTGGAACCGCAAGCGTATCAGCA
ACTGCGTCGCCGACTACTCTGTGCTGTACAACTCCGCTAGCTTCTCTACTTT
CAAGTGCTACGGCGTGTCACCTACCAAGCTGAACGACCTGTGCTTCACTAA
CGTCTACGCCGACTCCTTCGTGATCCGCGGAGACGAAGTCCGTCAGATCGC
TCCTGGACAGACCGGAAAGATCGCTGACTACAACTACAAGCTGCCAGACG
ACTTCACTGGCTGCGTGATCGCTTGGAACTCAAACAACCTGGACTCCAAG
GTCGGTGGCAACTACAACTACCTGTACAGGCTGTTCAGAAAGTCAAACCT
GAAGCCTTTCGAGCGCGACATCTCAACCGAAATCTACCAGGCTGGTTCCAC
TCCCTGCAACGGTGTGGAGGGCTTCAACTGCTACTTCCCCCTGCAGTCCTA
CGGTTTCCAGCCAACCAACGGAGTCGGTTACCAGCCTTACCGTGTGGTCGT
 
GCTGAGCTTCGAACTGCTCCACGCTCCTGCTACTGTGTGCGGTCCCAAGAA
GTCTACTAACCTGGTCAAAAACAAATGTGTCAACTTCAACTTCAACGGTCA
CCACCACCACCACCACCACCACTGATAAGCTT
SEQ ID No.9 (Insect cell signal peptide + escherichia coli Trx (thioredoxin) + S protein RBD corresponding nucleotide sequence): 
GGATCCATGCTGCTGGTCAACCAGAGCCACCAGGGCTTCAACAAGGAACA
CACTTCCAAGATGGTGTCCGCCATCGTCCTGTACGTGCTGCTGGCCGCCGC
TGCTCACAGCGCTTTCGCCGCTGACAGCGACAAGATCATCCACCTGACTGA
CGACAGCTTCGACACTGACGTGCTGAAGGCTGACGGTGCTATCCTGGTCG
ACTTCTGGGCCGAGTGGTGCGGCCCTTGCAAGATGATCGCTCCCATCCTGG
ACGAGATCGCCGACGAGTACCAGGGTAAACTGACTGTGGCCAAGCTGAAC
ATCGACCAGAACCCCGGTACTGCTCCTAAGTACGGCATCCGTGGTATCCCC
ACTCTGCTGCTGTTCAAGAACGGTGAGGTGGCCGCTACCAAGGTCGGTGC
TCTGAGCAAGGGCCAGCTGAAGGAGTTCCTGGACGCTAACCTGGCTGGTT
CCGGCAGCGGCCACATGCACCACCACCATCACAGCAGCGGCGACGAC
GACGACAAGGTGCAGCCAACCGAATCTATCGTCAGATTCCCAAACATCACT
AACCTGTGCCCTTTCGGAGAGGTGTTCAACGCTACCAGGTTCGCCAGCGTC
TACGCTTGGAACCGCAAGCGTATCAGCAACTGCGTCGCCGACTACTCTGTG
CTGTACAACTCCGCTAGCTTCTCTACTTTCAAGTGCTACGGCGTGTCACCTA
CCAAGCTGAACGACCTGTGCTTCACTAACGTCTACGCCGACTCCTTCGTGA
TCCGCGGAGACGAAGTCCGTCAGATCGCTCCTGGACAGACCGGAAAGATC
GCTGACTACAACTACAAGCTGCCAGACGACTTCACTGGCTGCGTGATCGCT
TGGAACTCAAACAACCTGGACTCCAAGGTCGGTGGCAACTACAACTACCT
GTACAGGCTGTTCAGAAAGTCAAACCTGAAGCCTTTCGAGCGCGACATCT
CAACCGAAATCTACCAGGCTGGTTCCACTCCCTGCAACGGTGTGGAGGGC
TTCAACTGCTACTTCCCCCTGCAGTCCTACGGTTTCCAGCCAACCAACGGA
GTCGGTTACCAGCCTTACCGTGTGGTCGTGCTGAGCTTCGAACTGCTCCAC
GCTCCTGCTACTGTGTGCGGTCCCAAGAAGTCTACTAACCTGGTCAAAAAC
AAATGTGTCAACTTCAACTTCAACGGT TAA AAGCTT
SEQ ID No.10 (Insect cell signal peptide + insect Trx (thioredoxin) + S protein RBD corresponding nucleotide sequence): 
GGATCCATGCTGCTGGTCAACCAGAGCCACCAGGGTTTCAACAAGGAACA
CACCAGCAAGATGGTGAGCGCTATCGTGCTGTACGTCCTGCTGGCCGCTGC
TGCTCACAGCGCTTTCGCTGCTGACTCCATCCACATCAAGGACAGCGACGA
CCTGAAGAACCGTCTGGCCGAGGCCGGTGACAAGCTGGTCGTCATCGACT
TCATGGCCACTTGGTGCGGTCCTTGCAAGATGATCGGCCCTAAGCTGGACG
AGATGGCTAACGAGATGTCCGACTGCATCGTGGTCCTGAAGGTGGACGTCG
ACGAGTGCGAGGACATCGCCACCGAATACAACATCAACAGCATGCCCACC
TTCGTGTTCGTGAAGAACAGCAAGAAGATCGAGGAATTTTCCGGCGCTAA
CGTCGACAAGCTGCGTAACACCATCATCAAGCTGAAGCTGGCCGGCTCCG
GCTCCGGCCACATGCATCACCACCACCACCATTCCTCCGGTGACGACGACG
ACAAGGTGCAGCCAACCGAATCTATCGTCAGATTCCCAAACATCACTAACC
TGTGCCCTTTCGGAGAGGTGTTCAACGCTACCAGGTTCGCCAGCGTCTACG
CTTGGAACCGCAAGCGTATCAGCAACTGCGTCGCCGACTACTCTGTGCTGT
ACAACTCCGCTAGCTTCTCTACTTTCAAGTGCTACGGCGTGTCACCTACCA
AGCTGAACGACCTGTGCTTCACTAACGTCTACGCCGACTCCTTCGTGATCC
GCGGAGACGAAGTCCGTCAGATCGCTCCTGGACAGACCGGAAAGATCGCT
GACTACAACTACAAGCTGCCAGACGACTTCACTGGCTGCGTGATCGCTTGG
AACTCAAACAACCTGGACTCCAAGGTCGGTGGCAACTACAACTACCTGTA
CAGGCTGTTCAGAAAGTCAAACCTGAAGCCTTTCGAGCGCGACATCTCAA
CCGAAATCTACCAGGCTGGTTCCACTCCCTGCAACGGTGTGGAGGGCTTCA
ACTGCTACTTCCCCCTGCAGTCCTACGGTTTCCAGCCAACCAACGGAGTCG
GTTACCAGCCTTACCGTGTGGTCGTGCTGAGCTTCGAACTGCTCCACGCTC
CTGCTACTGTGTGCGGTCCCAAGAAGTCTACTAACCTGGTCAAAAACAAAT
GTGTCAACTTCAACTTCAACGGT TAA AAGCTT
SEQ ID No.11, including restriction enzyme cutting site BglII (1-6) + EK restriction enzyme cutting site (7-21) + S-RBD(aa320-545) optimized by Escherichia coli biased codons + restriction enzyme cutting site Xho I (last 6 bits): 
AAGCTTGACGACGACGACAAGGTGCAGCCGACCGAAAGCATTGTGCGCTT
TCCGAACATTACCAACCTTTGTCCTTTCGGTGAGGTATTCAATGCAACACGC
 
TTTGCTTCAGTTTATGCTTGGAACCGCAAACGCATTTCAAACTGTGTTGCTG
ATTATTCAGTTCTTTATAACTCAGCTTCATTCTCCACCTTTAAATGTTATGGC
GTTTCACCTACAAAGCTGAATGATCTTTGTTTCACCAATGTTTATGCTGATTC
ATTTGTTATTCGCGGCGATGAAGTTCGCCAGATTGCTCCTGGCCAGACAGG
CAAGATAGCCGATTATAACTATAAACTTCCTGATGATTTCACGGGATGTGTTA
TTGCTTGGAACTCAAACAACCTTGATTCAAAGGTCGGTGGCAACTATAACT
ATCTTTATCGCCTGTTCCGGAAGTCAAACCTTAAACCTTTCGAGAGAGATAT
TTCAACAGAAATTTATCAGGCTGGCTCAACACCTTGTAACGGCGTTGAAGG
CTTTAACTGTTATTTCCCACTGCAAAGCTATGGCTTTCAGCCTACAAACGGC
GTTGGCTATCAGCCTTATCGCGTTGTTGTTCTTTCATTTGAACTTCTTCATGC
TCCTGCTACAGTTTGTGGCCCTAAGAAAAGCACTAATCTGGTGAAAAACAA
ATGTGTGAACTTTAACTTTAACGGCTGATAACTCGAG
SEQ ID No.12, including restriction enzyme cutting site XhoI (1-6) + signal peptide cleavage site (7-21) + S-RBD(aa320-545) optimized by Escherichia coli biased codons + restriction enzyme cutting site Xba I (last 6 bits): 
CTCGAGAAAAGAGTTCAACCTACAGAATCAATCGTTAGATTTCCTAACATC
ACAAACCTTTGTCCTTTCGGCGAGGTCTTCAATGCCACAAGATTTGCATCA
GTTTATGCATGGAACAGAAAGCGTATATCAAACTGTGTTGCAGATTATTCAG
TTCTTTATAACTCAGCATCATTCTCTACCTTTAAATGTTATGGAGTTTCACCT
ACAAAGCTCAATGATCTTTGTTTCACTAATGTTTATGCAGATTCATTTGTTAT
CAGAGGAGATGAAGTTAGACAAATCGCACCTGGACAAACAGGAAAGATTG
CCGATTATAACTATAAACTTCCTGATGATTTCACCGGCTGTGTTATCGCATGG
AACTCAAACAATCTCGACAGCAAAGTAGGTGGGAATTACAATTACTTGTAC
CGGCTATTTAGGAAGTCCAACCTCAAGCCGTTCGAGCGCGATATCTCAACA
GAAATCTATCAAGCAGGATCAACACCTTGTAACGGAGTTGAAGGATTTAAC
TGTTATTTCCCGCTACAATCATATGGATTTCAACCTACAAACGGAGTTGGATA
TCAACCTTATAGAGTTGTTGTTCTTTCATTTGAACTTCTTCATGCACCTGCA
ACAGTTTGTGGACCTAAGAAGTCTACGAACCTTGTTAAGAATAAGTGTGTT
AACTTTAACTTTAACGGATGATAATCTAGA
SEQ ID No.13 (human IL6 protein signal peptide + 8xHis signal peptide + EK restriction enzyme cutting site + RBD 320-545 226aa):

ATGAACAGCTTCAGCACCAGCGCCTTCGGCCCCGTGGCCTTCAGCCTGGGC
CTGCTGCTGGTGCTGCCCGCCGCCTTCCCCGCCCCCCACCACCACCACCAC
CACCACCACGACGACGACGACAAGGTGCAGCCCACCGAGAGCATCGTGA
GGTTCCCCAACATCACCAACCTGTGCCCCTTCGGCGAGGTGTTCAACGCCA
CCAGGTTCGCCAGCGTGTACGCCTGGAACAGGAAGAGGATCAGCAACTGC
GTGGCCGACTACAGCGTGCTGTACAACAGCGCCAGCTTCAGCACCTTCAA
GTGCTACGGCGTGAGCCCCACCAAGCTGAACGACCTGTGCTTCACCAACG
TGTACGCCGACAGCTTCGTGATCAGGGGCGACGAGGTGAGGCAGATCGCC
CCCGGCCAGACCGGCAAGATCGCCGACTACAACTACAAGCTGCCCGACGA
CTTCACCGGCTGCGTGATCGCCTGGAACAGCAACAACCTGGACAGCAAGG
TGGGCGGCAACTACAACTACCTGTACAGGCTGTTCAGGAAGAGCAACCTG
AAGCCCTTCGAGAGGGACATCAGCACCGAGATCTACCAGGCCGGCAGCAC
CCCCTGCAACGGCGTGGAGGGCTTCAACTGCTACTTCCCCCTGCAGAGCTA
CGGCTTCCAGCCCACCAACGGCGTGGGCTACCAGCCCTACAGGGTGGTGG
TGCTGAGCTTCGAGCTGCTGCACGCCCCCGCCACCGTGTGCGGCCCCAAG
AAGAGCACCAACCTGGTGAAGAACAAGTGCGTGAACTTCAACTTCAACGG
CTGA

The present invention provides a recombinant vector, which contains the polynucleotide.

Furthermore, the recombinant vector is at least one of the insect baculovirus expression vector, mammalian cell expression vector, Escherichia coli expression vector and yeast expression vector.

Preferably, the insect baculovirus expression vector is pFastBac1.

Preferably, the Escherichia coli expression vector is pET32a.

Preferably, the yeast expression vector is pPICZaA.

Preferably, the mammalian cell expression vector is a CHO cell expression vector.

Furthermore, the CHO cell expression vector is preferably pTT5 or FTP-002.

The present invention provides a host cell, which contains the recombinant vector.

Furthermore, the hose cell is at least one of the insect cell, mammalian cell, escherichia coli, and yeast.

Preferably, the insect cell is at least one of the sf9 cell, sf21 cell, and Hi5 cell.

Preferably, the mammalian cell is a CHO cell.

The present invention provides the preparation method for the protein, which comprises the following step: culturing the host cell to express the protein or precursor and then recovering the protein.

The present invention provides the preparation method for the protein, which comprises the following step: constructing the recombinant vector containing the polynucleotide to realize human immunity and thus generating the protein.

Furthermore, the vector is at least one of the mRNA, DNA vaccine, adenovirus, vaccinia Ankara virus, and adeno-associated virus.

The present invention provides the anti-SARS-CoV-2-infection protein and vaccine, particularly the S protein targeted at the SARS-CoV-2 virus, which particularly block the ACE2 receptor-binding domain of the S protein to induce the production of antibodies in the body for immunoreaction and block the binding the SARS-CoV-2 S protein and the ACE2 receptor of the host cell, thus helping the host to fight against the corona virus infection.

### Brief Description of the Drawings

Fig. 1 shows the results of affinity test for the protein disclosed in the present invention and the ACE2 protein in Test Example 1;
Fig. 2 shows the results of antibody titer test in Test Example 2;
Fig. 3 shows the results of response between the antibody in the patient with SARS-CoV-2 and the protein disclosed in the present invention as determined by ELISA in Test Example 3;
Fig. 4 shows the test results of blocking the binding between the RBD protein and ACE2 receptor in Test Example 4;
Fig. 5 shows the results of neutralizing antibody detection in Test Example 5;
Fig. 6 shows the results of copy detection for virus gRNA and sgRNA in lung tissue in Test Example 5;
Fig. 7 shows the results of copy detection for virus gRNA and sgRNA in throat swab in Test Example 5;
Fig. 8 shows the results of copy detection for virus gRNA and sgRNA in anal swab in Test Example 5;
Fig. 9 shows the section staining results for the lung tissue in Test Example 5;
Fig. 10 shows the results of mouse challenge experiment against SARS-CoV-2 infection in Test Example 6;
Fig. 11 shows the detection results of cytokines INF-γ and IL-4 in Test Example 7;
Fig. 12 shows the results of cytokine level detection in Test Example 8;
Fig. 13 is a schematic diagram for extracellular domain composition of the SARS-CoV-2 S protein;
Fig. 14 is a spectrogram of Escherichia coli expression vector pET32a in Embodiment 3;
Fig. 15 is a spectrogram of yeast expression vector pPICZaA in Embodiment 4;

### Detailed Description of the Preferred Embodiments

The technical solution of the present invention will be described in combination with the embodiments. Those skilled in the art will understand that the following embodiments are used only to describe the present invention, but not to limit the scope of the present invention. It should be noted that, where no specific technologies or conditions are indicated in the embodiments of the present invention, the technologies or conditions described by the literature in the present art or specified in the product specification shall apply. The reagents or apparatuses, where no specific manufacturer is indicated, are all commercially available conventional products.

S protein is a glycosylated protein and preferably, the insect baculovirus expression system or mammalian cell expression system (CHO expression system) is used for better gaining the natural S protein. The specific preparation method is described as bellow:

### Embodiment 1 Preparing the anti-SARS-CoV-2-infection protein disclosed in the present invention by use of the insect baculovirus expression system

**Vector construction:** Recombinant proteins produced by use of the insect baculovirus expression system mainly utilize the S protein receptor-binding domain (RBD). SARS-CoV-2 S protein is a protein located on the virus envelope. In order to simulate the secretion process of the SARS-CoV-2 S protein, a GP67 signal peptide is added to the N-terminal during the construction of S protein RBD to facilitate the secretion expression of the protein. This signal peptide will be spontaneously excised by insect cells during the secretion process of the protein. At the same time, in order to facilitate purification and increase the water solubility of the protein, a thioredoxin tag and an enterokinase (EK) restriction enzyme cutting site are also introduced into the sequence. The complete nucleotide sequence is shown in SEQ ID No.8 or SEQ ID No.10. The expression vector of S protein RBD is constructed based on the pFastBac1 vector (amicillin resistance), BamHI and HindIII restriction enzyme cutting sites are inserted into the pFast-bacI vector, and escherichia coli biased codons are used for optimization.

**Amplification of recombinant baculovirus:** The Bac-to-Bac expression system is used to construct recombinant bacmids in Escherichia coli (DH10Bac, containing bacmid (kanamycin resistance) and the helper plasmid (tetracycin resistance)) by generating site-specific transposition through the Tn7 transposition element using the principle of bacterial transposon. The successfully recombined bacmids are extracted and transfected into sf9 insect cells with Cellfectin II to generate the recombinant baculoviruses that can express the target gene. The first generation of viruses are collected 72 h after the transfection, and then amplified from P2 to P4. P3 or P4 viruses are used to express the protein.

**Protein expression:** Hi5 insect cells (or sf9 and sf21 cells) are infected with the P3 or P4 viruses, with a multiplicity of infection (MOI) of 0.5-10, and the supernatant is collected after 48-72 hours of culture. The optimal harvest time may vary according to the amount of virus and cell status, and it is generally appropriate when about 50% of the cells get infected as observed by the microscopic examination.

**Protein purification:** The harvested culture supernatant is centrifuged at 4°C at a high speed and filtered with an 0.22 µm filter membrane. The recombinant protein is initially purified by the affinity purification method (Histrap nickel column). Then, the recombinant protein is further purified by the MonoQ ion column and Superdex 200 10/300GL molecular sieve. The protein purity is required to reach more than 95% as determined by the SDS-PAGE detection. The prepared protein is dissolved or diluted to 1-5 mg/ml with enzyme digestion buffer. A corresponding amount of enterokinase (EK enzyme) is added at a proportion of 1U enterokinase for 50 µg recombinant protein, mixed, and left standing for digestion at 25°C for 16 hours to remove the tag of recombinant protein. The nucleotide shown in SEQ ID No.8 is used to express the protein SEQ ID No.3, and the nucleotide shown in SEQ ID No.10 is used to express the protein SEQ ID No.4. The obtained recombinant protein can be used for subsequent studies, such as animal immunization.

### Embodiment 2 Preparing the anti-SARS-CoV-2-infection protein disclosed in the present invention by use of the CHO cell expression system

Recombinant protein vaccines produced by use of CHO cells are mainly targeted at the S protein receptor-binding domain (RBD). These fragments are genetically synthesized according to the codon preference, and polyhistidine is used as the purification tag (6His). The complete nucleotide sequence is shown in SEQ ID No. 13. Then, it is constructed into the high expression vector pTT5 and the expressed amino acid sequence is the precursor protein as shown in SEQ ID No.14.

### Embodiment 3 Expressing the anti-SARS-CoV-2-infection protein in the Escherichia coli

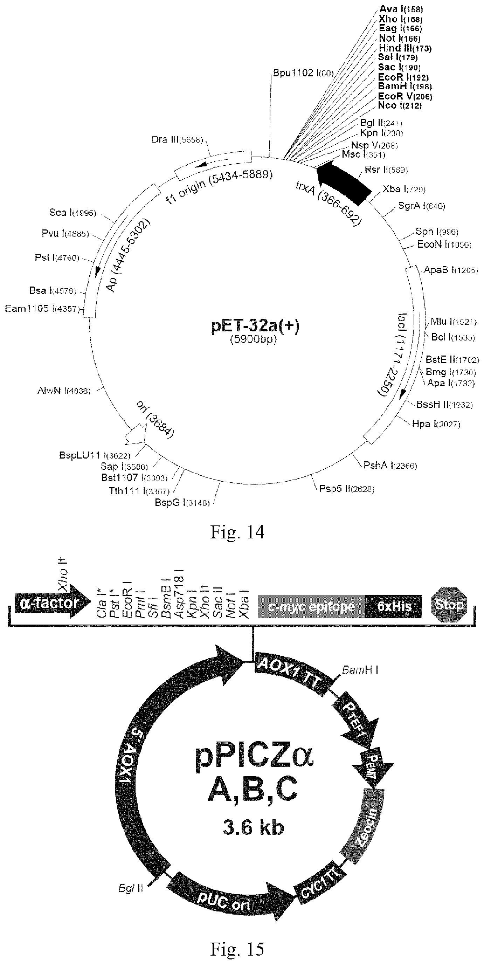

pET32a from Novagen is used as the expression vector (see the plasmid profile in Fig. 14), which contains a T7 promoter and where the transcription of downstream target genes is regulated by the IPTG The N-terminal of the expressed product is fused with thioredoxin (Trx) and purified by the metal chelate affinity chromatography (MCAC) in a single step. In order to remove Trx after purification of the target protein, the enterokinase (EK) restriction enzyme cutting site is added after Trx. The complete nucleotide sequence is shown in SEQ ID No.11.

The recombinant plasmids are expressed in Escherichia coli strain BL21 (DE3) respectively. The electrophoresis results show that the size of the obtained target protein is similar to the predicted size and verified by Western Blot. The content of the target protein is more than 30% of the total protein, mainly in the form of inclusion bodies. Under denaturing conditions, the protein can be purified by the metal chelate affinity chromatography (MCAC) in a single step to allow for a purity of higher than 95% and a yield of 200-400mg/L. The target protein can be renatured by dialysis, with a renaturation efficiency of higher than 50%.

To reduce the production cost, metal chelate columns can be substituted with reversed-phase columns for single-step purification, which can also produce the high-purity target protein with a purity of higher than 95%.

### Embodiment 4 Expressing the anti-SARS-CoV-2-infection protein in the yeast

The nucleotide sequence as shown in SEQ ID No.12 is cloned into the double enzyme (Xho I/Xba I) site of the yeast expression vector pPICZaA (Invitrogen; see the profile of yeast expression vector pPICZaA in Fig. 15) to secrete and express the protein using the factor a secretion signal in the methylotrophic yeast.

pPICZaA plasmid is used to mediate and integrate the S-RBD gene into the methylotrophic yeast chromosome, and then methanol is used to induce the expression of the target protein. The expressed target protein is present in a soluble form in the culture medium of the methylotrophic yeast, which could be purified in a single step by reverse-phase column chromatography, with the expression quantity reaching 200-400mg/L.

### Embodiment 5 Preparing the anti-SARS-CoV-2-infection vaccine

Antigens are prepared under sterile conditions and the purified recombinant protein antigens (prepared according to embodiments 1-4) are diluted with 5mmol/L phosphate buffer (pH7.2) to a concentration of 80 mcg/mL. Adjuvants are prepared under aseptic conditions and the aluminum hydroxide adjuvants (with a content of 14.55mg/mL) are diluted with 5mmol/L phosphate buffer (pH7.2) to a concentration of 2.0 mg/mL. Antigen-adjuvant adsorption is carried out under sterile conditions at a speed of 20 mL/min, the diluted protein antigen liquid is added dropwise to the diluted aluminum hydroxide adjuvant working solution at a volume ratio (V/V) of 1:1, so that the final concentration of recombinant protein antigens in the mixed solution is 40 mcg/mL, and the final concentration of aluminum adjuvants is 1.0 mg/mL. The reaction temperature is kept at 25°C and the stirring speed at 800rpm. After dropping, the adsorption is performed for 60min at the temperature of 25°C and the stirring speed of 800rpm. The pH of the mixed solution is adjusted to 7.2. The solution is stored at 4°C away from light. The adsorbed vaccine preparations are characterized, including particle size, site position, antigen content, adjuvant content, adsorption rate, pH value, endotoxin, adjuvant and antigen adsorption rate, adsorption strength and its hold status, and antigen integrity and stability after adsorption. For filling, the qualified vaccine preparations refilled into the 1mL sterile penicillin bottles or ampoule bottles in 1mL/vial. Continuous stirring is kept when filling to make the filled liquid even. The filled vials are capped immediately after filling, attached with the serial number labels, and stored at 4°C away from light.

The advantageous effects of the present invention are demonstrated by the following test examples.

### Test Example 1 Affinity test for the protein disclosed in present invention and the ACE2 protein by surface plasmon resonance (SPR) analysis

Surface plasmon resonance detection was performed using a macromolecular interactometer Biacore 8K (GE Healthcare, Sweden). The ACE2-Fc was pre-anchored on the surface of Sensor Chip Protein A chip with a capture response unit (RU) value of ∼100RU. For kinetic analysis, the RBD protein of the present invention, whose amino acid sequence is shown in SEQ ID No.3, was handled to pass through the chip surface with a concentration gradient (1, 2, 4, 6, 8, 16, 32 nM) diluted by a double equal proportion respectively, and another channel is set as the blank control group. Antigen dissociation was performed for 300 seconds using HBS-EP+ dissociation solution at a flow rate of 30 mL/min, followed by 60 seconds of chip regeneration using glycine solution with pH 1.5 as the regeneration solution. In this process, the binding constant (Kₐ) and dissociation constant (K_{d}) of ACE2-Fc antibody and RBD protein of the present invention were respectively detected, and their affinity (KD) was calculated.

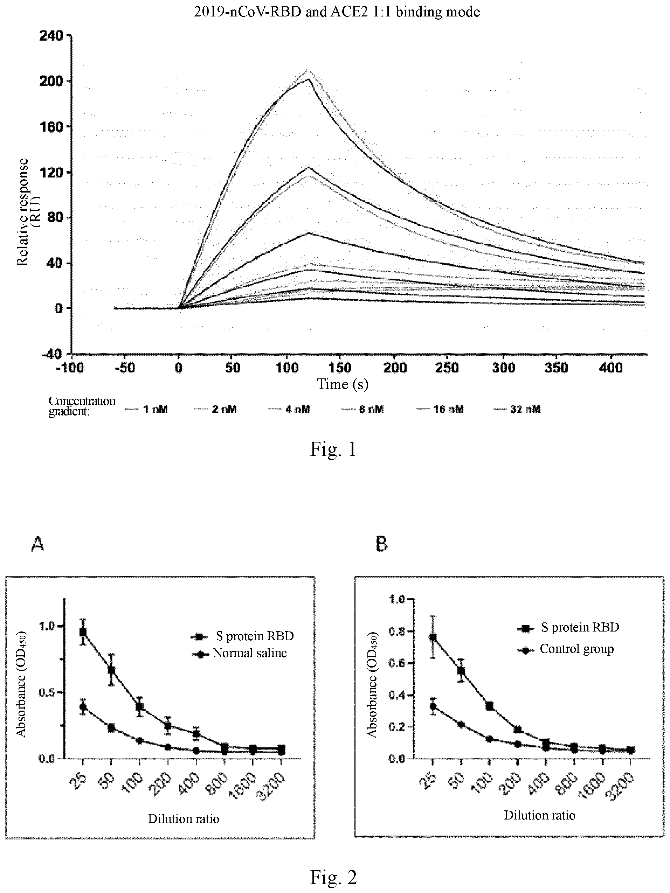

The results, as shown in Fig. 1, showed that the RBD protein of the present invention can bind with the ACE2 receptor protein efficiently and specifically, and suggested that the RBD protein of the present invention may maintain an integral spatial structure and have the same ACE2 receptor ability as the S protein RBD of the virus, providing a strong support for taking the in-vitro recombinant S protein RBD as vaccines. Its affinity K_{D} was 1.52×10⁻⁸ M (mol/L), dissociation constant K_{d} was 4.41×10⁻² (s⁻¹), and binding constant Ka was 3.85×10⁶ (Ms⁻¹).

### Test example 2 Inducing the RBD-specific antibodies in mice vaccinated with the vaccine disclosed in the present invention

**Animal immunization test:** BALB/c or C57BL/6 mice were injected with the recombinant proteins (with the amino acid sequence as shown in SEQ ID No.3) at doses ranging from 0.1 to 10.0µg per mouse; each group was assigned with five to ten mice. Each mouse was injected with a volume of 50µL of vaccine (prepared according to Embodiment 5) intramuscularly (im) in the right hind leg. Two immunization regimens were used: vaccination on days 1, 7, and 21, and vaccination on days 1, 14, and 21.

**Determination of mouse serum antibody by enzyme linked immunosorbent assay (ELISA):** On the 7th day after each immunization, the plasma of mice was collected by capillary orbital blood sampling from 5 mice in each group. After coagulating at room temperature for 1-2h, and centrifugation at 3000rpm/min for 10min at 4°C, the upper layer of serum was taken and stored at -20°C for later use. For the determination of serum IgG and subtype by the ELISA, a 1 µg/ml solution of recombinant protein S-Fc or RBD-Fc was prepared in 50 mM carbonate coating buffer (PH9.6), and added at 100 µl/well into a 96-well plate (Thermo Scientific, NUNC-MaxiSorp) for coating overnight at 4°C. For the preparation of 50mM carbonate coating buffer (PH9.6), 0.15g Na₂CO₃ and 0.293g NaHCO₃ were weighed and dissolved in double distilled water, the PH was adjusted to 9.6, then the volume was fixed to 100 ml and stored at 4°C for later use. The next day, the mice plasma was washed 3 times with PBS solution containing 0.1% Tween20 (PBST), blocked with blocking solution containing 1%BSA or 5% skim milk (prepared in PBST) for 1h at room temperature, and then washed once with PBST. The mice serum was diluted with blocking solution in different proportions, then added at 100µl well for incubation for 1h-2h at 37°C, and washed with PBST for 3 times. Then the plasma was added with HRP-goat anti-mouse IgG or HRP-anti-mouse IgG1, IgM or other subtype antibodies at 100 µ l/well (diluted in blocking solution at 1:5000), incubated at 37°C for 1h, and then washed with PBST for 5 times. Finally, 3,3',5,5' -tetramethylbiphenyl diamine (TMB) was added at 100µl/well, and after 10-15min of color development in the dark, 1M H2SO4 stop solution was added at 50µl/well, and the reading was performed on the microplate reader at 450nm wavelength after mixing. To prepare the 1 M H2SO4 stop solution, 2.7mL of concentrated sulfuric acid (98%) was added drop by drop to 47.3mL of double distilled water.

The test results are shown in Fig. 2. In order to measure the titer of RBD-specific antibody induced by the recombinant protein, serum was continuously diluted in different proportions and measured by titration, and the A450 optical density value was measured. As shown in Fig. 2, the recombinant protein vaccine elicited significant S protein RBD-specific antibodies. Serum collected 7 days after vaccination showed a strong antibody response, with IgG (Fig. 2A) and IgM (Fig. 2B) increased in different ratios, while the A450 optical density was significantly lower in the control group vaccinated with the normal saline, suggesting that the vaccine can rapidly induce immune responses and is important for the prevention of SARS-COV-2. These results indicated that the recombinant S protein RBD vaccine was highly immunogenic in mice.

### Test Example 3 Reaction determination between the antibody in the patient with SARS-CoV-2 and the protein disclosed in the present invention by ELISA

In this experiment, 16 serum samples from patients infected with SARS-CoV-2 were collected to investigate the immunogenicity of the RBD protein of the present invention in human bodies. ELISA was used for determination as follows:
A 96-well plate was coated with the RBD protein (with the amino acid sequence shown in SEQ ID No.3) at a concentration of 0.2µg/well, 100µl/ well, at 4°C overnight. A negative control well was set up during coating. The 96-well plate was removed the next day, and in half an hour after rewarming to the room temperature, the plate was washed with PBS for 4 times, 1 min each time. The 96-well plate was blocked with 1%BSA at 100µl/ well, incubated at 37°C for 30 min, and then washed again with PBS for 4 times, 1 min each time. The serum was diluted by 5 folds, namely adding 80µl of PBS for every 20µl of serum in each well. Then, the plate was incubated at 37°C for 30 min, washed with PBS for 4 times, 1 min each time, then added with the HRP-labeled secondary antibody (anti-human IgG/IgM antibody) diluted in a proportion of 1:2000 at 100ul/well, incubated at 37°C for 30 min, and washed with PBS for 4 times, 1 min each time. For color development, each well was added with 50µl of liquid A and then 50µl of liquid B and left standing at room temperature for 15 min. To stop, each well was added with 100µl of stop solution. Colorimetry with a microplate reader was conducted within 10 min. In this assay, for the detection of IgM antibodies, 15µl of serum in each well was added with 15µl PBS and then 150µl IgG adsorbent, and centrifuged at 10000rpm for 10 min; 100µl of supernatant was taken for detection.

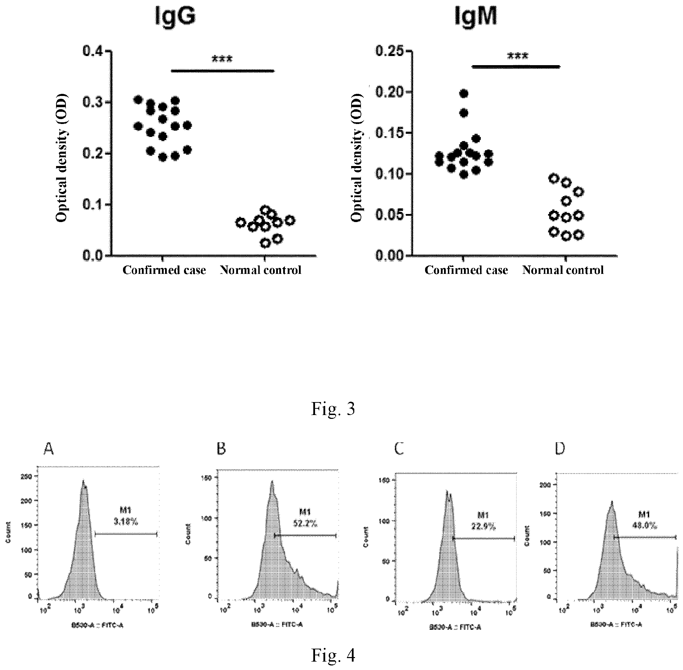

As shown in Fig. 3, serum from the 16 patients infected with SARS-CoV-2 had obvious response to the RBD protein of the present invention, and both IgM and IgG reactions were positive, while the serum from 10 healthy people showed negative reaction to the antigen, indicating that the SARS-CoV-2 S protein RBD had high immunogenicity as a vaccine in patients. The RBD protein prepared by the present invention can be recognized by the human immune system.

### Test Example 4 Blocking test for the binding between the RBD protein and ACE2 receptor

In this experiment, cell-expressed ACE2, a protein thought to retain its native conformation, was used to allow RBD binding activity to be measured by flow cytometry. Specific operations are as follows:
The in-vitro cultured cell strains with high expression of ACE2 (lung cancer A549) were digested and collected into flow cytometry tubes at 10⁶ cells/tube and washed with PBS/HBSS several times. Recombinant RBD-Fc protein at a final concentration of 1 ug/ml was added to each tube of cells and the serum from immunized anti-RBD mice was then added (after the mouse serum obtained from Test Example 2 was diluted by 50 folds) for incubation for 30 min at room temperature. For the positive control tube, no antiserum was added or normal serum from unimmunized mice was added. After washing with PBS/HBSS for several times, Anti-Human IgG (Fc specific)-FITC (SIGMA) fluorescent secondary antibody (1:100-1:200) was added for incubation at room temperature for 30 min in the dark. After washing with PBS/HBSS for several times and fixation by adding 500 µl PBS containing 1% paraformaldehyde, detection was performed by flow cytometry.

As shown in Fig. 4, the added RBD-Fc protein could significantly bind with the ACE2-expressing cells, while only background signal was detected if RBD-Fc protein was not added (negative control). Mouse antiserum effectively blocked the binding of the RBD-Fc protein with ACE2-expressing cells, while the unimmunized or pre-immunized serum of the same dilution showed no inhibitory activity.

### Test Example 5 Challenge experiment on non-human primates (such as rhesus monkey) with live SARS-CoV-2 virus

### 1. Experimental method

All research procedures involving nonhuman primates were reviewed and approved by the Institutional Animal Care and Use Committee of the Institute of Medical Biology, Chinese Academy of Medical Sciences, and were performed in the Animal BioSafety Level 4 (ABSL-4) facility at the National Kunming High-level Biosafety Primate Research Center in Yunan, China. The RBD protein used in this experiment was the protein of this prevention whose amino acid sequence is shown in SEQ ID No.4, and the vaccine was prepared according to Embodiment 5. Twelve nonhuman primates (rhesus monkeys) (aged 5-9 years) were used in live SARS-CoV-2 challenge experiments, and grouped as follows: (a) group 1 with 4 rhesus monkeys (n = 4), which were vaccinated with the vaccine comprising 40 µg RBD protein plus aluminum hydroxide adjuvant each dose; (b) group 2 with 3 rhesus monkeys (n = 3), which were vaccinated with the vaccine comprising 20 µg RBD protein plus aluminum hydroxide adjuvant each dose; (c) group 3 with 2 rhesus monkeys (n = 2), which was the normal saline control group; (d) group 4 with rhesus monkeys (n = 2), which was the aluminum hydroxide adjuvant control group. The nonhuman primates were immunized by intramuscular injection on days 0 and 7, followed by nasal challenge with SARS-CoV-2 (0.5 ml, 10⁶ pfu/ ml) 28 days after the initial immunization. To assess the neutralizing effect of SARS-CoV-2 infection, sera were collected on days 28 and 35 (5 days after virus vaccination) after the first immunization for neutralizing antibody assay. Vero E6 cells (5×10⁴/well) were inoculated in a 96-well plate and cultured overnight. SARS-CoV-2 with 100-fold TCID50 (50% tissue culture infective dose) was preincubated with an equal volume of diluted serum, and after incubation for 1 h at 37°C, the mixture was added to Vero E6 cells. On day 3 after infection, cytopathic effect (CPE) was recorded under a microscope, and neutralization titers were calculated for serum diluents producing EC 50 inhibition (50% neutralization). The control groups included the monkey serum treated with normal saline or aluminum hydroxide alone.

The contents of viral genomic RNA (gRNA) and viral subgenomic RNA (sgRNA, representing virus replication) were determined by quantitative real-time reverse transcription PCR (qRT-PCR). Viral loads in lung tissue, throat swabs, and anal swabs were determined by qRT-PCR based on sequences recommended by WHO and Chinese Center for Disease Control and Prevention, using primers and probes from the NP gene.
Forward: 5'-GGGGAACTTCTCCTGCTAGAAT-3' (SEQ ID No.15);
Reserve: 5'-CAGACATTTTGCTCTCAAGCTG-3' (SEQ ID No.16);
Probe: 5'-FAM-TTGCTGCTGCTTGACAGATT-TAMRA-3' (SEQ ID No.17)

According to the instructions for TaqMan Fast Virus 1-Step Master Mix (Article No.: 4444434), the reaction system was 10µL: 1 µL forward primer, 1 µL reverse primer, 0.25 µL probe, 2.5 µL mRNA template, 2.5 µL Master Mix, and 2.75 µL RNase-Free H2O. For PCR program setting and operation, operation instructions for BioRad CFX384 Real Time PCR System were consulted: reverse transcription (incubated at 25°C for 2 min and 50°C for 15 min); initiation (incubated at 95°C for 2 min); two-step amplification, 40 cycles (incubated at 95°C for 5s and 58°C for 31s).

The content of SARS-CoV-2 E gene subgenomic mRNA (sgmRNA) indicating virus replication was determined using the primer and probe with the following sequences:
Forward: 5'-GCTAGAGAACATCTAGACAAGAG-3' (SEQ ID No.18);
Reverse: 5'-ACACACGCATGACGACGTTATA-3' (SEQ ID No.19);
Probe: 5'-FAM-TGTGATCGGTAGGAATGACGCGAAGC-Quencher-3' (SEQ ID No.20);

The reaction system and PCR procedure were consistent with gRNA assay.

For paraffin embedding of slices, tissues were collected and fixed with 10% neutral formalin and embedded in paraffin. Sections were 5 µm thick and stained with hematoxylin and eosin (HE).

### 2. Experimental results

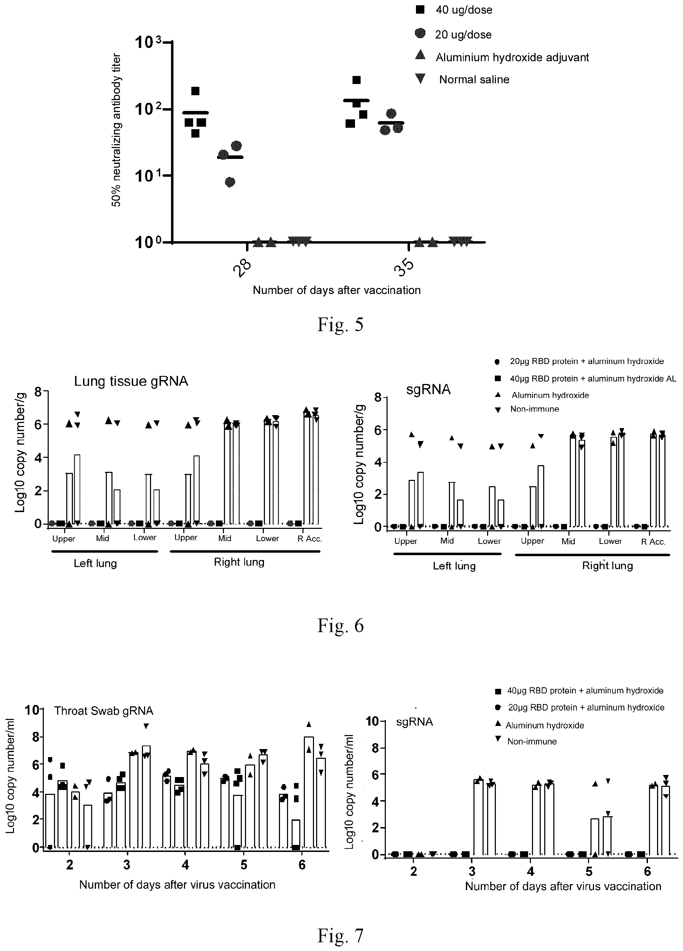

Neutralizing antibodies against live SARS-CoV-2 were detected in all vaccinated nonhuman primates but not in either control group, as shown in Fig. 5.

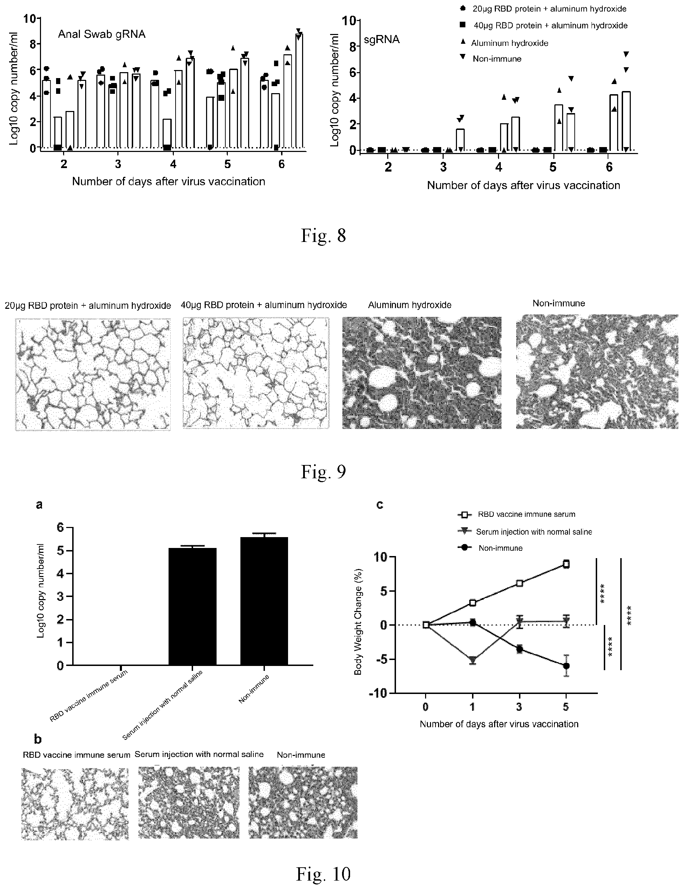

Quantitative real-time reverse transcription PCR (qRT-PCR) was used to detect viral genomic RNA (gRNA) and viral subgenomic RNA (sgRNA, representing virus replication). Lung tissues from nonhuman primates were collected on day 7 after challenge to assess virus replication status. The lung tissue of control group (normal saline group and aluminum hydroxide adjuvant group) showed excessive copies of viral gRNA and sgRNA. In contrast, there was no detectable virus replication in the groups vaccinated with 20 or 40 µg RBD protein plus adjuvant (Fig. 6). In addition, peak viral gRNA loads in throat swabs were observed 3 days after vaccination in the control groups (normal saline group and aluminum hydroxide adjuvant group), and these viral peaks could be blocked with vaccine, while the viral loads were only 1.6 and 3.8 percent per million in the 20 µg and 40 µg vaccine groups. Importantly, no detectable sgRNA was observed in throat swabs after viral challenge in both the 20 and 40 µg vaccine groups, whereas a high amount of sgRNA was observed in the control groups (normal saline group and aluminum hydroxide adjuvant group), indicating virus replication (Fig. 7). On days 5 and 6 after inoculation, peak viral gRNA and gRNA loads in anal swabs were observed in the control groups (normal saline group and aluminum hydroxide adjuvant group), while only an extremely low level was detected in the vaccinated groups, without detectable sgRNA in the anal swabs of nonhuman primates vaccinated with the 20 µg and µg vaccine (Fig. 8). The above results indicate that vaccination with the RBD protein vaccine of the present invention can prevent SARS-CoV-2 infection.

Lung tissues from the two control groups (normal saline group and aluminum hydroxide adjuvant group) showed the histopathologic changes typical of SARS-CoV-2 viral interstitial pneumonia, a key feature of COVID-19. As shown in Fig. 9, the alveolar walls were significantly thickened as observed under the microscope, and a large number of interstitial mononuclear inflammatory cells infiltrated. There were also numerous inflammatory infiltrates and serous exudates in the alveolar space, accompanied by the recognizable loss of lung tissue structures. In addition, diffuse bleeding and type II pneumonocyte hyperplasia were observed. In contrast, nonhuman primates vaccinated with the RBD protein (20µg or 40µg) showed no significant histopathologic changes and had a normal lung tissue appearance.

### Test Example 6 Mice challenge experiment against SARS-CoV-2 infection

BALB/c or C57BL/6 mice aged between 6 to 8 weeks were immunized by intramuscular injection of the recombinant RBD protein vaccine (i.e., the protein with amino acid sequence as shown in SEQ ID No.3; the vaccine prepared according to Embodiment 5) at different doses (0.1-20µg each). For example, mice received an injection on day 0, serum was collected on day 7, and mice in the control group were injected with either aluminum hydroxide immune adjuvant or normal saline alone. Serum was collected again on day 7 after immunization. The serum was stored at 4°C for used in the later experiment. The SPF hACE2 transgenic mice established by the Institute of Laboratory Animal Science, Chinese Academy of Medical Sciences and Peking Union Medical College were used in animal experiments of SARS-CoV-2 infection. Seven days after the first vaccination, 0.8ml of serum was collected. Serum from vaccine-immunized mice was used as the experimental group, and normal serum from normal saline treated mice was used as the control group. One day before SARS-CoV-2 virus challenge (intranasal infection, 10⁵ TCID50), hACE2 transgenic mice were injected with the serum intraperitoneally. In addition, mice infected with the virus but not received the serum injection served as controls. Five days after the virus challenge, the mice were killed and their lungs and other organs were harvested. Lung tissue was used to detect viral replication or fixed with 10% buffered formalin solution for histopathologic analysis. Real-time quantitative reverse transcriptase polymerase chain reaction (qRT-PCR) was performed with PowerUp SYBG Green Master Mix Kit (Applied Biosystems, USA) to determine viral RNA copy number in lung tissues of mice challenged with SARS-COV-2, expressed in the RNA copy number/ml of lung tissue. The primer sequence used for qRT-PCR was the envelope (E) gene against SARS-cov-2 as follows:
Forward: 5'-TCGTTTCGGAAGAGACAGGT-3' (SEQ ID No.21);
Reverse: 5'-GCGCAGTAAGGATGGCTAGT-3' (SEQ ID No.22).

The slices were stained with hematoxylin and eosin, and the histopathological changes were observed under the light microscope.

This experiment tested whether early humoral immunity through vaccination can prevent mice from being infected with the SARS-CoV-2 virus. Human ACE-2 transgenic mice were challenged with the SARS-CoV-2 virus, and lung tissues of the mice were collected 5 days after virus challenge to measure the virus replication status of the serum receiving immunization (the serum 7 days after the first immunization) or the control serum. As shown in Fig. 10, no viral replication was detected by quantitative real-time reverse transcriptase polymerase chain reaction (qRT-PCR) in mice treated with the immune serum induced by the RBD protein vaccine, whereas the level of viral replication was higher in lung tissues of the control mice. Accordingly, the lung tissues of the control mice showed significant interstitial pneumonia histopathological changes, including significant alveolar wall thickening, extensive interstitial monocyte and lymphocyte infiltration, embolism, and serum exudate in the alveolar space. In contrast, no histopathological changes or slight exudation were observed in mice treated with the serum from mice immunized with the recombinant RBD protein vaccine. In addition, mice treated with the immune serum gained a slight amount of weight (approximately 8%) during the first 5 days after infection, while no weight gain in the control group and an 8% weight loss in the untreated group were observed. This experiment further confirmed that the antibody induced by the RBD protein vaccine could completely block the virus infection.

### Test Example 7 Induction of cellular immune response by the RBD protein vaccine disclosed in the present invention

BALB/c or C57BL/6 mice aged between 6 to 8 weeks were immunized by intramuscular injection of the recombinant RBD protein vaccine (i.e., the protein with amino acid sequence as shown in SEQ ID No.3; the vaccine prepared according to Embodiment 5) at different doses (0.1-20µg each). For example, mice received an injection on day 0, serum was collected on day 7, and mice in the control group were injected with either aluminum hydroxide immune adjuvant or normal saline alone. Spleen T lymphocytes were collected again 7 days after immunization. To investigate the cellular immune response, mice immunized with S protein RBD or PBS were killed and their lymphocytes were extracted for IL-4 and IFN-γ detection by ELISA. In brief, mouse spleen lymphocytes (1×10⁶/mL) were cultured in RPMI 1640 medium (containing 10% fetal bovine serum, 100U/mL penicillin, 100µg/mL streptomycin, 1mM pyruvate, 50µM β-mercaptoethanol, 20U/mL IL-2). At the same time, 1µg/mL RBD protein was added for culture and stimulation for 72 hours. Cells without RBD protein stimulation were used as the negative control. The supernatant was collected for ELISA.

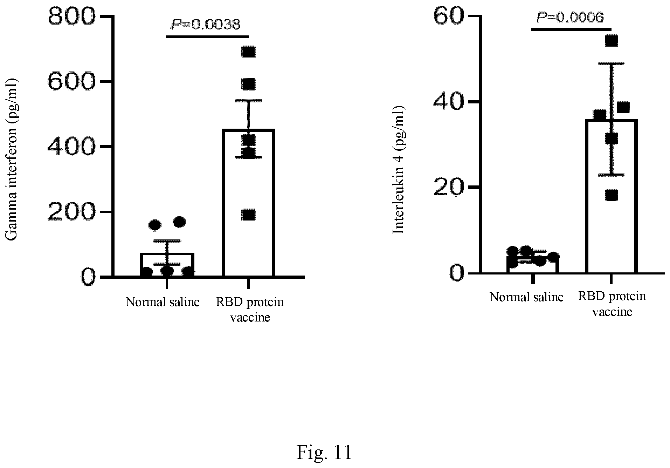

Because cellular immune responses may play a role in clearing SARS-CoV-2 infection, in which both CD4 and CD8 positive T cells are involved in immune responses against SARS virus infection, the potential cellular immune responses to the vaccine were also examined. Lymphocytes were collected on day 7 after the first vaccination, and cytokines produced by the lymphocytes such as INF-γ (gamma interferon) and IL-4 (interleukin-4) were measured by ELISA. Stimulation of the isolated mouse lymphocytes with the recombinant RBD protein showed that vaccine-immunized mice produced more IFN-γ and IL-4 in the lymphocytes, whereas only IFN-γ and IL-4 at the background level were detected in mice treated with normal saline after the lymphocytes were stimulated by the recombinant RBD protein (Fig. 11).

### Test Example 8 Safety experiment for the vaccine disclosed in the present invention

Mice were immunized with the vaccine of the present invention. BALB/c or C57BL/6 mice aged between 6 to 8 weeks were immunized by intramuscular injection of the recombinant RBD protein vaccine (i.e., the protein with amino acid sequence as shown in SEQ ID No.3; the vaccine prepared according to Embodiment 5) at different doses (0.1-20µg each). No pathological changes were found in heart, brain, liver, spleen, lung, kidney and other organs. No changes in blood cells or blood biochemical indicators were found. For example, this experiment measured the cytokine level in the blood to see whether vaccination caused changes in cytokines in the systemic inflammatory response. Mice received a single injection on day 0, serum was collected on day 7, and control mice received aluminum hydroxide immune adjuvant or normal saline alone. On the 7th day after the first inoculation, serum was collected from the mice, and the cytokines of TNF-a, IFN-γ, IFN-a, IFN-b, IL-6 and IL-4 were detected by ELISA. Cytokine content in the serum was determined using Thermo Fisher Scientific-eBioscience Elisa kit as follows: the antigen was coated, diluted with coating buffer at 200 µL/well, sealed, and incubated overnight at 4°C; the coating solution was discarded after coating, and the plate was washed three times with washing buffer at 250 µL/well or higher; 200 µL ELISA/ELISPOT diluent (1×) was used for blocking for 1 h at room temperature; the standard was prepared according to the concentration requirements in the specification, and the standard with the highest concentration was diluted by two-fold dilution method, a total of 8 points, and ELISA/ELISPOT diluent (1×) was used as a control; the serum was diluted with ELISA/ELISPOT diluent (1×) for later use; after the plate was washed, the plate was added with 100µL of standards and samples to be tested, sealed and incubated for 2 h at room temperature; the antibody to be detected was diluted with ELISA/ELISPOT diluent (1×), the plate was washed 3-5 times, the diluted antibody to be detected was added to each well at 100 µL/well, and the plate was sealed and incubated for 1 hour at room temperature; Hrp-labeled antibodies were diluted with ELISA/ELISPOT diluent (1×), and the plate was washed 3-5 times, the diluted antibiotin protein HRP was added to each well at 100 µL/well, and the plate was sealed and incubated for 30 min at room temperature; the plate was washed 5-7 times, 1X TMB solution was added to each well at 100 µL/well, and color development was performed for 10-15 min at room temperature; stop solution was added at 100 µL/well for termination; plate reading was carried out with the detection wavelength being 450nm and the reference wavelength being 570nm.

The experimental results showed that the no difference in the blood cytokine level was found between vaccine-immunized mice and control mice (aluminum hydroxide immunized adjuvant or normal saline only) (see Fig. 12). The above experimental results indicated that vaccination with the vaccine may not cause systemic inflammatory reaction.

It is important to note that the specific characteristics, structures, materials or features described in this specification may be combined in any one or more embodiments in a suitable manner. In addition, as long as no mutual contradiction is caused, those skilled in the art may incorporate and combine the different embodiments described in this specification and the characteristics of the different embodiments.

## Claims

1. An anti- SARS-CoV-2-infection protein, **characterized by** containing a domain that binds with the angiotensin-converting enzyme 2 (ACE2) receptor as contained in the SARS-CoV-2 S protein.

2. The protein according to claim 1, **characterized in that** the amino acid sequence of the domain is SEQ ID No.1 or SEQ ID No.2.

3. The protein according to claim 1, **characterized in that** the amino acid sequence is at least one of the SEQ ID No.1, SEQ ID No.2, SEQ ID No.3 and SEQ ID No.4.

4. The precursor of the protein according to any one of claims 1 to 3, **characterized by** linking the anti- SARS-CoV-2-infection protein with a signal peptide and/or protein tag; preferably, the protein tag is at least one of the histidine tag, thioredoxin tag, glutathione transferase tag, ubiquitin-like modified protein tag, maltose-binding protein tag, c-Myc protein tag, Avi tag protein tag, and nitrogen source utilization substance A protein tag.

5. The precursor according to claim 4, **characterized by** also linking the anti-SARS-CoV-2-infection protein with a protease recognition sequence for protein tag removal; preferably, the protease is at least one of the enterokinase, TEV protease, thrombin, coagulation factor Xa, carboxypeptidase A, and rhinovirus 3c protease.

6. The precursor according to claim 4 or claim 5, **characterized in that** the amino acid sequence is at least one of the SEQ ID No.5, SEQ ID No.6, SEQ ID No.7 and SEQ ID No.14.

7. The use of the protein according to any one of claims 1 to 3 and/or the precursor according to any one of claims 4 to 6 in preparing the SARS-CoV-2 infection prevention and/or treatment drugs.

8. The vaccine for SARS-CoV-2 infection prevention and/or treatment, **characterized by** comprising the protein according to any one of claims 1 to 3 and/or the precursor according to any one of claims 4 to 6, as well as the pharmaceutically acceptable excipient or auxiliary ingredient.

9. The vaccine according to claim 8, **characterized in that** the auxiliary ingredient includes immunologic adjuvant; preferably, the immunologic adjuvant is at least one of the aluminum salt, calcium salt, plant saponin, plant polysaccharide, monophosphate-lipid A, murinyl dipeptide, murinyl tripeptide, squalene oil-in-water emulsion, bacterial toxin, GM-CSF cytokine, lipid, and cationic liposome material.

10. The vaccine according to claim 9, **characterized by** satisfying at least one of the following specifications: the aluminum salt is at least one of the aluminum hydroxide and alum; the calcium salt is tricalcium phosphate; the plant saponin is QS - 21 or ISCOM; the plant polysaccharide is astragalus polysaccharide; the squalene oil-in-water emulsion is MF59; the bacterial toxin is at least one of the recombinant cholera toxin and diphtheria toxin; the lipid is at least one of the phosphatidyl ethanolamine, phosphatidyl choline, cholesterol, and dioleyl phosphatidyl ethanolamine; the cationic liposome material is at least one of the (2,3-Dioleoyloxy-propyl)-trimethylammonium-chloride, N-[1-(2, 3-dioleoxy chloride) propyl]-N,N,N-trimethylamine chloride, cationic cholesterol, trifluoroacetic acid dimethyl-2, 3-dioleoxy propyl-2-(2-spermine formyl amino) ethyl ammonium, dodecyl trimethyl ammonium bromide, tetradecyl trimethyl ammonium bromide, cetyl-methyl-ammoniumbromide, dimethyldioctadecylammonium bromide (DDAB), and CpG ODN.

11. The vaccine according to any one of claims 8 to 10, **characterized in that** the vaccine is an injection preparation; preferably, the vaccine is an intramuscular injection preparation.

12. The polynucleotide, **characterized by** encoding the protein according to any one of claims 1 to 3 and/or the precursor according to any one of claims 4 to 6.

13. The polynucleotide according to claim 12, **characterized in that** the nucleotide sequence is at least one of the SEQ ID No.8, SEQ ID No.9, SEQ ID No.10, SEQ ID No.11, SEQ ID No.12 and SEQ ID No.13.

14. The recombinant vector, **characterized by** comprising the polynucleotide according to claim 12 or claim 13.

15. The recombinant vector according to claim 14, **characterized in that** at least one of the insect baculovirus expression vector, mammalian cell expression vector, Escherichia coli expression vector and yeast expression vector is used; preferably, the insect baculovirus expression vector is pFastBacl; preferably, the Escherichia coli expression vector is pET32a; preferably, the yeast expression vector is pPICZaA; preferably, the mammalian cell expression vector is a CHO cell expression vector; furthermore, the CHO cell expression vector is preferably pTT5 or FTP-002.

16. The host cell, **characterized by** containing the recombinant vector according to claim 14 or claim 15.

17. The host cell according to claim 16, **characterized in that** at least one of the insect cell, mammalian cell, Escherichia coli, and yeast is used; preferably, the insect cell is at least one of the sf9 cell, sf21 cell, and Hi5 cell; preferably, the mammalian cell is a CHO cell.

18. The preparation method for the protein according to any one of claims 1 to 3, **characterized by** comprising the following step: culturing the host cell according to claim 16 or claim 17 to express the protein or precursor and then recovering the protein.

19. The preparation method for the protein according to any one of claims 1 to 3, **characterized by** comprising the following step: constructing the recombinant vector containing the polynucleotide according to claim 12 or claim 13 to realize human immunity and thus generating the protein.

20. The preparation method according to claim 19, **characterized in that** the vector is at least one of the mRNA, DNA vaccine, adenovirus, vaccinia Ankara virus, and adeno-associated virus.
